# EUROPEAN PATENT APPLICATION

(11) **EP 4 755 987 A1**
(43) Date of publication of application: **10.06.2026**
(21) Application number: 24851664.3
(22) Date of filing: 29.07.2024
(51) Int. Cl.: C09J 175/04, A61F 13/0246, C08G 18/10, C08G 18/22, C08G 18/48, C08G 18/64, C09J 7/38, C09J 11/06, C09J 11/08, C09J 175/08

(54) **ADHESIVE AGENT COMPOSITION, METHOD FOR PRODUCING SAME, AND ADHESIVE AGENT**

(30) Priority: 04.08.2023 JP 2023128100
(71) Applicant: AGC Inc., Chiyoda-ku Tokyo 100-8405 (JP)
(72) Inventor: SASAZAKI, Hiroki, Tokyo 100-8405 (JP); KOMINE, Takuya, Tokyo 100-8405 (JP); NAKAMURA, Makito, Tokyo 100-8405 (JP)
(74) Representative: Müller-Boré & Partner Patentanwälte PartG mbB
(86) International application number: PCT/JP2024/027021
(87) International publication number: WO 2025/033239

(57) **Abstract**

An adhesive composition comprising a hydroxyl-terminated urethane prepolymer, a tackifier resin, and a polyisocyanate compound, in which the hydroxyl-terminated urethane prepolymer is a reaction product of an oxyalkylene polymer having a number-average molecular weight of 3,000 or more, and a diisocyanate compound.

## Description

### Technical Field

The present invention relates to an adhesive composition to obtain an adhesive agent applicable to paste on the skin, a method for producing the same, an adhesive agent, a patch, a wearable device and a wearable device kit.

### Background Art

Urethane-based adhesive agents have been employed as adhesive agents for skin patches, such as adhesive plaster, surgical tapes, poultices, and plaster agents, and adhesive agents used for pasting a patch-type wearable device on the skin to continuously obtain biometrics such as body temperature and electroencephalogram.

As the urethane-based adhesive agents for pasting on the skin, for example, PTL1 describes a medical adhesive composition including: (A) a urethane resin; (B) a tackifier resin having a softening point of 75°C or higher and having a hydroxy group; and (C) an isocyanate curing agent, the composition having an adhesive force ratio [(X2)/(X1)] of an adhesive force (X2) to a stainless steel plate after 24 hours in a 23°C, 50% relative humidity (RH) environment to an adhesive force (X1) to a stainless steel plate after 20 minutes in a 23°C, 50% RH environment of 0.8 to 1.5.

### Citation List

### Patent Literature

PTL 1: JP 2020-81438 A

### Summary of Invention

### Technical Problem

However, as a result of investigations by the present inventors, the adhesive composition described in PTL1 was found to have insufficient properties to maintain an applied state in a wet condition with water for a long time, i.e., insufficient long-term water resistance.

The present invention has been made in consideration of these circumstances, and aims to provide an adhesive composition that can provide an adhesive agent having a sufficient adhesive force to a device and a sealant and sufficient long-term water resistance, a method for producing the same, as well as an adhesive agent, a patch, a wearable device, and a wearable device kit.

### Solution to Problem

The present inventors have found, as a result of extensive investigations, that the adhesive composition described in Examples of PTL1 is incapable of providing sufficient long-term water resistance when the number-average molecular weight of the raw material polyol for the urethane resin is as small as 2,000 or less. The present inventors have also found that an adhesive composition containing a urethane resin synthesized using a polyol with a large number-average molecular weight and containing a tackifier resin can provide an adhesive agent capable of providing sufficient adhesion to a device and a sealant, and having sufficient long-term water resistance. The present invention was accomplished based on these findings.

The present invention provides the following means.
[1] An adhesive composition comprising a hydroxyl-terminated urethane prepolymer, a tackifier resin, and a polyisocyanate compound, in which the hydroxyl-terminated urethane prepolymer is a reaction product of an oxyalkylene polymer having a number-average molecular weight of 3,000 or more, and a diisocyanate compound.
[2] The adhesive composition according to [1], in which the hydroxyl-terminated urethane prepolymer is a reaction product of an oxyalkylene polymer having a number-average molecular weight of 3,000 or more, and a diisocyanate compound in the presence of a tin-free catalyst.
[3] The adhesive composition according to [1] or [2], in which the tackifier resin has a softening point of 70°C or higher.
[4] The adhesive composition according to any of [1] to [3], in which the tackifier resin is a terpene-based tackifier resin or a styrene-based tackifier resin.
[5] The adhesive composition according to any of [1] to [4], in which the hydroxyl-terminated urethane prepolymer has a content of structural units based on ethylene oxide of 8 to 55% by mass.
[6] The adhesive composition according to any of [1] to [5], in which the oxyalkylene polymer comprises an oxyalkylene polymer A having the number of hydroxyl groups per molecule of 2 or more and an oxyalkylene polymer B having the number of hydroxyl groups per molecule of 1.
[7] The adhesive composition according to any of [1] to [6], in which the hydroxyl-terminated urethane prepolymer has a weight-average molecular weight of 50,000 or more.
[8] A method for producing an adhesive composition by reacting an oxyalkylene polymer having a number-average molecular weight of 3,000 or more, and a diisocyanate compound to obtain a hydroxyl-terminated urethane prepolymer, and
   mixing the hydroxyl-terminated urethane prepolymer, a tackifier resin, and a polyisocyanate compound.
[9] The method for producing an adhesive composition according to [8], in which the hydroxyl-terminated urethane prepolymer is a reaction product of an oxyalkylene polymer having a number-average molecular weight of 3,000 or more, and a diisocyanate compound in the presence of a tin-free catalyst.
[10] The method for producing an adhesive composition according to [9], in which the tin-free catalyst is more than 0.001 parts by mass and 0.1 parts by mass or less per 100 parts by mass of the oxyalkylene polymer.
[11] An adhesive agent, being a cured product of the adhesive composition according to any of [1] to [7].
[12] A patch comprising a substrate and an adhesive agent layer provided on at least a part of a surface of the substrate, in which the adhesive agent layer comprises the adhesive agent according to [11].
[13] The patch according to [12], being an adhesive tape.
[14] A wearable device comprising a wearable device main body and an adhesive agent layer, in which
   the adhesive agent layer is provided on at least a part of the surface of the wearable device facing the skin and comprises the adhesive agent according to [11].
[15] A wearable device kit comprising a wearable device main body, and at least any of the adhesive agent according to [11] and the patch according to [12] or [13].

### Advantageous Effects of Invention

According to the present invention, an adhesive composition that can provide an adhesive agent having sufficient adhesion to a device and a sealant and sufficient long-term water resistance, a method for producing the same, as well as an adhesive agent, a patch, a wearable device, and a wearable device kit can be provided.

### Description of Embodiments

The definitions and meanings of the terms and notations described herein are shown in the followings.

The skin refers to human skin.

The number-average molecular weight (Mn) and weight-average molecular weight (Mw) are polystyrene equivalent molecular weights determined by measuring with gel permeation chromatography (GPC) based on calibration curves created by using standard polystyrene samples.

The hydroxyl value is determined by measurement in accordance with JIS K 1557-1:2007.

The degree of unsaturation is determined by measurement in accordance with JIS K 1557-3:2007.

The isocyanate index indicates an equivalent ratio of isocyanate groups to be reacted and active hydrogen-containing groups (e.g., hydroxyl group) ([isocyanate group]/[active hydrogen-containing groups]), expressed as percentage.

### [Adhesive Composition]

The adhesive composition according to an embodiment of the present invention (hereinafter, referred to as the present embodiment) contains a hydroxyl-terminated urethane prepolymer, a tackifier resin, and a polyisocyanate compound, in which the hydroxyl-terminated urethane prepolymer is a reaction product of an oxyalkylene polymer having a number-average molecular weight of 3,000 or more, and a diisocyanate compound.

According to the adhesive composition of the present embodiment, an adhesive agent having a sufficient adhesive force to a device and a sealant and sufficient long-term water resistance can be obtained.

### (Hydroxyl-Terminated Urethane Prepolymer)

The hydroxyl-terminated urethane prepolymer of the present embodiment is a reaction product of an oxyalkylene polymer having a number-average molecular weight of 3,000 or more, and a diisocyanate compound. Accordingly, an adhesive agent having a sufficient adhesive force to a device and a sealant, and sufficient long-term water resistance can be obtained.

The hydroxyl-terminated urethane prepolymer of the present embodiment is preferably a reaction product of an oxyalkylene polymer having a number-average molecular weight of 3,000 or more, and a diisocyanate compound in the presence of a tin-free catalyst. This makes it possible to obtain an adhesive agent that is skin friendly and does not contain any tin-containing catalysts having adverse effects on the skin.

The weight-average molecular weight of the hydroxyl-terminated urethane prepolymer is preferably 50,000 or more, more preferably 100,000 or more, and further preferably 200,000 or more, from the viewpoint of obtaining an adhesive agent having a sufficient adhesive force to a device and a sealant and sufficient long-term water resistance. The weight-average molecular weight is preferably 50,000 to 500,000, more preferably 100,000 to 500,000, further preferably 200,000 to 450,000, yet further preferably 200,000 to 400,000, and still further preferably 250,000 to 300,000, from the viewpoint of obtaining an adhesive agent having a sufficient adhesive force to a device and a sealant and sufficient long-term water resistance, and ease of production.

The content of structural units based on ethylene oxide (hereinafter, abbreviated as "EO units") in the hydroxyl-terminated urethane prepolymer is preferably 8 to 55% by mass, more preferably 8 to 40% by mass, further preferably 8 to 20% by mass, yet further preferably 8 to 18% by mass, still further preferably 8 to 16% by mass, and even still further preferably 8 to 13% by mass, from the viewpoint of obtaining an adhesive agent having a sufficient adhesive force to a device and a sealant and sufficient long-term water resistance.

The lower the content of EO units in the hydroxyl-terminated urethane prepolymer, the lower the skin irritation, and when the content falls within the above range, it is easy to obtain an adhesive agent being skin friendly, and having a sufficient adhesive force to a device and a sealant, and sufficient long-term water resistance.

The EO units in the hydroxyl-terminated urethane prepolymer are derived from EO units constituting the oxyalkylene chains of the oxyalkylene polymer. The content of EO units in the hydroxyl-terminated urethane prepolymer can be determined by analyzing the monomer composition of the oxyalkylene chains in the hydroxyl-terminated urethane prepolymer through measurement of ¹³C-NMR (nuclear magnetic resonance).

The total amount of structures derived from the oxyalkylene polymer having a number-average molecular weight of 3,000 or more, and structures derived from the diisocyanate compound in the hydroxyl-terminated urethane prepolymer is preferably 80 to 100% by mass, more preferably 90 to 100% by mass, and further preferably 95 to 100% by mass.

### <Oxyalkylene Polymer>

The number-average molecular weight of the oxyalkylene polymer according to the present embodiment is 3,000 or more. When the hydroxyl-terminated urethane prepolymer is obtained using an oxyalkylene polymer having a small molecular weight, it is difficult to increase the molecular weight of the hydroxyl-terminated urethane prepolymer, and therefore, the water resistance is low. Since the oxyalkylene polymer according to the present embodiment has a number-average molecular weight of 3,000 or more, a hydroxyl-terminated urethane prepolymer having a high molecular weight can be obtained, and thereby an adhesive agent having a sufficient adhesive force to a device and a sealant and sufficient long-term water resistance can be obtained.

Further, the number-average molecular weight of the oxyalkylene polymer according to the present embodiment is preferably 50,000 or less. When it is 50,000 or less, it is possible to prevent the molecular weight of the obtained hydroxyl-terminated urethane prepolymer from being too high, thereby preventing the viscosity of the adhesive composition from becoming too high, and resulting in the adhesive composition being excellent in handling.

From these viewpoints, the number-average molecular weight of the oxyalkylene polymer is preferably 3,000 to 50,000, more preferably 5,500 to 45,000, further preferably 6,000 to 40,000, yet further preferably 7,000 to 30,000, and still further preferably 8,000 to 25,000.

The oxyalkylene polymer may be one oxyalkylene polymer, or two or more oxyalkylene polymers.

When a plurality of oxyalkylene polymers are used in the synthesis of the hydroxyl-terminated urethane prepolymer, the number-average molecular weight of the oxyalkylene polymers means the weight-averaged value based on the amounts of the plurality of the oxyalkylene polymers blended used in the synthesis of the hydroxyl-terminated urethane prepolymer.

When a plurality of oxyalkylene polymers are used in the synthesis of the hydroxyl-terminated urethane prepolymer, the number-average molecular weight of at least one of the oxyalkylene polymers needs to be 3,000 or more; however, the number-average molecular weight of all the oxyalkylene polymers may be 3,000 or more. For example, when the number of the oxyalkylene polymers used in the synthesis of the hydroxyl-terminated urethane prepolymer are three, namely, an oxyalkylene polymer (1), an oxyalkylene polymer (2), and an oxyalkylene polymer (3), the number-average molecular weight of at least one of these three (e.g., oxyalkylene polymer (1)) needs to be 3,000 or more; however, the number-average molecular weight of the two of these three (e.g., oxyalkylene polymers (1) and (2)) may be 3,000 or more, or the number-average molecular weight of all of the three (i.e., all of the oxyalkylene polymers (1), (2), and (3)) may be 3,000 or more. Provided that the weight-averaged value of the number-average molecular weight of all (three) of the oxyalkylene polymers (1), (2), and (3) based on the amounts blended must be 3,000 or more.

The content of the one having a number-average molecular weight of 3,000 or more is preferably 60 to 100% by mass, more preferably 80 to 100% by mass, and further preferably 90 to 100% by mass, and may be, for example, 100% by mass, out of a total of 100% by mass of the plurality of oxyalkylene polymers used in the synthesis of the hydroxyl-terminated urethane prepolymer.

The average content of EO units in the oxyalkylene polymer is preferably 8 to 55% by mass. When the average content of EO units in the oxyalkylene polymer falls within the above range, it is easy to obtain an adhesive agent having good adhesive force that has a low load on the skin and exhibits good fixation of a circuit or the like. From the viewpoint, the average content of EO units in the oxyalkylene polymer is more preferably 8 to 40% by mass, further preferably 8 to 20% by mass, yet further preferably 8 to 18% by mass, still further preferably 8 to 16% by mass, and even still further preferably 8 to 13% by mass.

The average content of EO units in the oxyalkylene polymer means the weight-averaged value of the contents of EO units in each oxyalkylene polymer based on the amounts of a plurality of the oxyalkylene polymers blended used in the synthesis of the hydroxyl-terminated urethane prepolymer. The content of EO units in each of the oxyalkylene polymers is a value calculated based on the amount of EO blended in the raw material in the synthesis of the oxyalkylene polymers. Note that the content of EO units in the oxyalkylene polymer can also be determined through measurement of ¹³C-NMR, in the same manner as the content of EO units in the hydroxyl-terminated urethane prepolymer.

The lower the average content of EO units in the oxyalkylene polymer, the lower the skin irritation, and when the average content falls within the above range, it is easy to obtain an adhesive agent being skin friendly, and having a sufficient adhesive force to a device and a sealant and sufficient long-term water resistance.

The oxyalkylene polymer has an average number of hydroxyl groups per molecule of preferably 1.5 to 3.0, more preferably 1.7 to 2.8, and further preferably 1.9 to 2.6, from the viewpoint of obtaining an adhesive agent having a sufficient adhesive force to a device and a sealant and sufficient long-term water resistance. The average number of hydroxyl groups per molecule of the oxyalkylene polymer means the weight-averaged value based on the amounts of a plurality of the oxyalkylene polymers blended used in the synthesis of the hydroxyl-terminated urethane prepolymer.

The oxyalkylene polymer may be one oxyalkylene polymer, or two or more oxyalkylene polymers.

The oxyalkylene polymer preferably comprises an oxyalkylene polymer A having the number of hydroxyl groups per molecule of 2 or more (hereinafter, also simply referred to as "polymer A") and an oxyalkylene polymer B having the number of hydroxyl groups per molecule of 1 (hereinafter, also simply referred to as "polymer B").

It is preferable that the hydroxyl groups of the polymer A and the polymer B react with the isocyanate group of the diisocyanate compound to form a urethane bond, and unreacted hydroxyl groups correspond to terminal hydroxyl groups of the molecular chain of the hydroxyl-terminated urethane prepolymer.

The total content of the polymer A and the polymer B in the oxyalkylene polymer is preferably 80% by mass or more, more preferably 90% by mass or more, further preferably 95% by mass or more, and yet further preferably 100% by mass.

The content ratio of the polymer A and the polymer B in the oxyalkylene polymer is preferably 10/90 to 95/5, more preferably 30/70 to 90/10, further preferably 50/50 to 85/15, and yet further preferably 60/40 to 85/15 at a mass ratio, from the viewpoint of obtaining an adhesive agent having a sufficient adhesive force to a device and a sealant and sufficient long-term water resistance.

### [Oxyalkylene Polymer A]

The number of hydroxyl groups per molecule of the polymer A is 2 or more, and preferably 2 to 6.

The polymer A may be one oxyalkylene polymer, or two or more oxyalkylene polymers. When the polymer A consists of two or more oxyalkylene polymers, good adhesive force of the adhesive agent is easily adjusted.

When the polymer A consists of two or more oxyalkylene polymers, the average number of hydroxyl groups per molecule of the polymer A is preferably 2.1 to 3.0, more preferably 2.2 to 2.9, and further preferably 2.3 to 2.8.

When the average number of hydroxyl groups of the polymer A falls within the above range, it is easy to obtain an adhesive agent that suppresses a load on the skin where a device is fixed, and is less likely to leave adhesive residues on the skin.

The average number of hydroxyl groups per molecule of the polymer A can be calculated by the hydroxyl value of the polymer A and the measured value of Mn, using the formula of the hydroxyl value × Mn/56,100.

When the polymer A consists of two or more oxyalkylene polymers, examples of the polymer A include an oxyalkylene polymer A1 having the number of hydroxyl groups per molecule of 3 (hereinafter, also simply referred to as "polymer A1"), and an oxyalkylene polymer A2 having the number of hydroxyl groups per molecule of 2 (hereinafter, also simply referred to as "polymer A2"). One polymer A1 may be used alone, or two or more may be used. Similarly, one polymer A2 may be used alone, or two or more may be used.

The polymer A can contain an oxyalkylene polymer having the number of hydroxyl groups per molecule of 4.

Note that when the polymer A consists of the polymer A1 and the polymer A2, the weight-averaged value of the number of hydroxyl groups per molecule of 3 of the polymer A1 and the number of hydroxyl groups per molecule of 2 of the polymer A2 based on the composition ratio (amount blended) of the polymer A1 and the polymer A2 can be considered as the average number of hydroxyl groups of the polymer A.

Out of a total of 100 parts by mass of the polymer A, the total amount of the polymer A1 and the polymer A2 accounts for preferably 85 parts by mass or more, more preferably 90 parts by mass or more, and further preferably 95 parts by mass or more, from the viewpoint of ease of adjustment and the like of the adhesive force of the adhesive agent.

Out of a total of 100 parts by mass of the polymer A, it is particularly preferably that the total amount of the polymer A1 and the polymer A2 account for 100 parts by mass, that is, the polymer A consist of the polymer A1 and the polymer A2.

From the viewpoint of obtaining an adhesive agent having a sufficient adhesive force to a device and a sealant and sufficient long-term water resistance, the polymer A1 accounts for preferably 20 parts by mass or more, more preferably 25 to 95 parts by mass, and further preferably 30 to 90 parts by mass, out of a total of 100 parts by mass of the polymer A1 and the polymer A2.

From the similar viewpoint, the polymer A2 accounts for preferably 80 parts by mass or less, more preferably 5 to 75 parts by mass, and further preferably 10 to 70 parts by mass, out of a total of 100 parts by mass of the polymer A1 and the polymer A2.

When the polymer A consists of, for example, the polymer A1 and the polymer A2, the content of the polymer A1 accounts for preferably 10 to 60 parts by mass, more preferably 20 to 50 parts by mass, and further preferably 30 to 50 parts by mass, out of a total of 100 parts by mass of the polymer A1, the polymer A2, and the polymer B in the oxyalkylene polymer, from the viewpoint of obtaining an adhesive agent having a sufficient adhesive force to a device and a sealant and sufficient long-term water resistance.

From the similar viewpoint, the content of the polymer A2 accounts for preferably 15 to 50 parts by mass, more preferably 30 to 50 parts by mass, and further preferably 30 to 40 parts by mass, out of a total of 100 parts by mass of the polymer A1, the polymer A2, and the polymer B in the oxyalkylene polymer.

From the similar viewpoint, the content of the polymer B accounts for preferably 5 to 40 parts by mass, more preferably 10 to 35 parts by mass, and further preferably 10 to 20 parts by mass, out of a total of 100 parts by mass of the polymer A1, the polymer A2, and the polymer B in the oxyalkylene polymer.

The average content of EO units in the polymer A is preferably 0 to 80% by mass, more preferably 1 to 50% by mass, further preferably 5 to 30% by mass, and yet further preferably 8 to 25% by mass.

The average content of EO units in the polymer A can be determined by analyzing the monomer composition of oxyalkylene chains in the polymer A through the measurement of ¹³C-NMR. For example, when the oxyalkylene chains in the polymer A consist of EO units and PO units, the content of EO units can be calculated based on the area of the peak showing methylene groups of the EO units and the area of the peak showing methyl groups of the PO units.

When the polymer A consists of, for example, the polymer A1 and the polymer A2, the same applies to the respective content of EO units in the polymer A1 and the polymer A2.

Note that the average content of EO units in the polymer A can be considered as a value obtained by calculating from the amount of EO blended in a raw material for synthesis of the polymer A. When the polymer A consists of, for example, the polymer A1 and the polymer A2, the weight-averaged value of the content of EO units of the polymer A1 and the content of EO units of the polymer A2 based on the composition ratio (amount blended) of the polymer A1 and the polymer A2 can be considered as the content of EO units of the polymer A.

The polymer A has Mn of preferably 1,000 to 50,000, more preferably 5,000 to 30,000, and further preferably 8,000 to 25,000, from the viewpoint of formation of an adhesive agent layer having good adhesive force, suppression of adhesive residues, good flexibility, and the like of the adhesive agent.

Mw/Mn (molecular weight distribution) of the polymer A is closed to 1, and preferably 1.25 or less, more preferably 1.22 or less, and further preferably 1.20 or less, since the narrower the molecular weight distribution, the less likely the hydroxyl-terminated urethane prepolymer becomes high viscous, leading to increased synthesis efficiency.

In addition, the degree of unsaturation of the polymer A is preferable to be closer to 0 meq/g, preferably 0.020 meq/g or less, more preferably 0.018 meq/g or less, and further preferably 0.015 meq/g or less, from the viewpoint of good curability of the hydroxyl-terminated urethane prepolymer, suppression of adhesive residues of the adhesive agent, and the like.

When the polymer A consists of two or more oxyalkylene polymers, each of the oxyalkylene polymers preferably falls within the above Mn, Mw/Mn, and degree of unsaturation.

The synthesis method of the polymer A is not particularly limited, and for example, the polymer A can be obtained by ring-opening addition polymerization of a compound having a cyclic ether structure, preferably alkylene oxide, to an initiator having two or more active hydrogens in the presence of a catalyst. When the polymer A consists of the polymer A1 and the polymer A2, a method can be used in which the ring-opening addition polymerization of a compound having a cyclic ether structure is carried out by concurrently using an initiator having three active hydrogens and an initiator having two active hydrogens to simultaneously generate the polymer A1 and the polymer A2. From the viewpoint of adjusting the amount of the polymer A1 and the polymer A2 blended more precisely, a method is preferable in which the polymer A obtained by the ring-opening addition polymerization of a compound having a cyclic ether structure to an initiator having three active hydrogens and the polymer B obtained by the ring-opening addition polymerization of a compound having a cyclic ether structure to an initiator having two active hydrogens are separately synthesized and then mixed together.

The compound having a cyclic ether structure is preferably linear or branched having 2 to 14 carbon atoms, more preferably 2 to 10 carbon atoms, and further preferably 2 to 4 carbon atoms. The compound having a cyclic ether structure may be used alone, or in combination of two or more.

Examples of the compound having a cyclic ether structure include ethylene oxide (EO), propylene oxide (PO), 1,2-butylene oxide, 2,3-butylene oxide, methyl glycidyl ether, butyl glycidyl ether, 2-ethylhexyl glycidyl ether, lauryl glycidyl ether, hexyl glycidyl ether, and tetrahydrofuran. Among these, EO and PO are preferable.

When two or more compounds having a cyclic ether structure are used in combination, for the polymer A, the sequence of oxyalkylene groups derived from each of the compounds may be random or blocked.

Examples of the group having an active hydrogen in the initiator include hydroxyl groups, carboxy groups, and amino groups having a hydrogen atom bound to a nitrogen atom. Among these, the hydroxyl group is preferable, and the alcoholic hydroxyl group is more preferable.

Examples of the initiator having three hydroxyl groups include glycerin, trimethylolethane, trimethylolpropane, and 1,2,6-hexanetriol. Any one of these may be used alone, or two or more may be used in combination.

Examples of the initiator having two hydroxyl groups include ethylene glycol, diethylene glycol, propylene glycol, and dipropylene glycol. Any one of these may be used alone, or two or more may be used in combination.

The number of active hydrogens of the initiator generally corresponds to the number of hydroxyl groups per molecule of the oxyalkylene polymer. The polymer A1 can be synthesized, for example, by using glycerin as the initiator. Also, the polymer A2 can be synthesized, for example, by using propylene glycol as the initiator.

Note that the type and amount of the initiator used as a raw material for synthesis of the oxyalkylene polymer can be identified through the measurement of ¹³C-NMR. When the polymer A consists of the polymer A1 and the polymer A2, the ratio of the polymer A1 to the polymer A2 is determined by determining the content ratio of the oxyalkylene groups, such as the EO units and PO units, bound to each initiator, from the information on the type and amount of the initiator for the polymer A through the measurement of ¹³C-NMR.

The ring-opening addition polymerization can be performed by using a known catalyst, for example, alkali catalysts such as potassium hydroxide, transition metal compound-porphyrin complex catalysts such as a complex obtained by reacting an organic aluminum compound with porphyrin, double metal cyanide complex catalysts, and catalysts consisting of a phosphazene compound. Among these catalysts, the double metal cyanide complex (DMC) catalyst is preferable, since an oxyalkylene polymer having narrow molecular weight distribution and relatively low viscosity is easily obtained. As the double metal cyanide complex include, a known compound can be used, and examples thereof include a zinc hexacyanocobaltate complex with tert-butanol as the ligand.

The synthesis of the oxyalkylene polymer by the ring-opening addition polymerization using a DMC catalyst can be performed by a known method, and for example, a production method described in WO 2003/062301, WO 2004/067633, JP 2004-269776 A, JP 2005-15786 A, WO 2013-065802, and JP 2015-10162 A can be applied.

### [Oxyalkylene Polymer B]

The number of hydroxyl groups per molecule of the oxyalkylene polymer B is 1. The polymer B may be one oxyalkylene polymer, or two or more oxyalkylene polymers.

The number of hydroxyl groups of the polymer B can be confirmed by identifying the type and amount of the initiator used as a raw material through the measurement of ¹³C-NMR as in the case of the polymer A.

The polymer B has Mn of preferably 4,000 to 30,000, more preferably 4,500 to 25,000, and further preferably 5,000 to 20,000, from the viewpoint of formation of an adhesive agent layer having good flexibility, good adhesive force, suppression of adhesive residues, and the like of the adhesive agent.

Mw/Mn of the polymer B is closed to 1, and preferably less than 1.20, more preferably less than 1.13, and further preferably less than 1.10, since the narrower the molecular weight distribution, the less likely the hydroxyl-terminated urethane prepolymer becomes high viscous, leading to increased synthesis efficiency.

In addition, the degree of unsaturation of the polymer B is preferably 0.015 meq/g or less, more preferably 0.013 meq/g or less, and further preferably 0.010 meq/g or less, and it is preferable to be closer to 0 meq/g, from the viewpoint of good curability of the hydroxyl-terminated urethane prepolymer, suppression of adhesive residues of the adhesive agent layer, and the like.

The synthesis method of the polymer B is not particularly limited, and for example, the polymer B can be obtained by ring-opening addition polymerization of a compound having a cyclic ether structure, preferably alkylene oxide, to an initiator having one active hydrogen in the presence of a catalyst.

The compound having a cyclic ether structure is similar to those described for the polymer A. PO alone, or EO and PO in combination is preferable.

The polymer B preferably contains an average content of EO units of 0 to 80% by mass, more preferably 0 to 60% by mass, and further preferably 0 to 50% by mass, from the viewpoint of obtaining an adhesive agent having a good balance between the adhesive force to the skin and the adhesive force to a device, and its crystallinity being suppressed.

The average content of EO units in the polymer B can be determined as in the case of the polymer A.

The group having an active hydrogen in the initiator includes those similar to the case of the polymer A, and the hydroxyl group is preferable, and the alcoholic hydroxyl group is more preferable.

The initiator having one hydroxyl group is preferably a monohydric alcohol having 2 to 4 carbon atoms, from the viewpoint of ease of availability, and the like; examples thereof include n-propanol, isopropanol, n-butanol, sec-butanol, isobutanol, and tert-butanol, and among these, n-butanol, sec-butanol, isobutanol, and tert-butanol are preferable. One initiator may be used alone, or two or more may be used in combination.

The ring-opening addition polymerization can be performed by a known method as in the case of the polymer A.

### <Diisocyanate Compound>

The diisocyanate compound used for synthesis of the hydroxyl-terminated urethane prepolymer is an organic compound having 2 isocyanate groups in a molecule. The diisocyanate compound may be an aliphatic diisocyanate compound, an alicyclic diisocyanate compound, or an aromatic diisocyanate compound. One diisocyanate compound may be used alone, or two or more may be used in combination.

An aliphatic diisocyanate compound may be either linear or branched, and examples thereof include tetramethylene diisocyanate, hexamethylene diisocyanate (HDI), dodecamethylene diisocyanate, 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate.

As an alicyclic diisocyanate compound, examples thereof include isophorone diisocyanate (IPDI), hydrogenated xylylene diisocyanate, 4,4'-dicyclohexylmethane diisocyanate (HMDI), 1,4-cyclohexane diisocyanate, methylcyclohexylene diisocyanate, and 1,3-bis(isocyanatomethyl)cyclohexane.

As a diisocyanate compound with an aromatic ring structure, examples thereof include tolylene diisocyanate, 2,2'-diphenylmethane diisocyanate, 2,4'-diphenylmethane diisocyanate, 4,4'-diphenylmethane diisocyanate (MDI), 4,4'-dibenzyl diisocyanate, 1,5-naphthylene diisocyanate, 1,3-phenylene diisocyanate, 1,4-phenylene diisocyanate, dialkyl diphenylmethane diisocyanate, tetraalkyl diphenylmethane diisocyanate, xylylene diisocyanate, and tetramethylxylylene diisocyanate (TMXDI).

Among these, HDI, IPDI, HMDI, and TMXDI are preferable, and HDI is more preferable from the viewpoint of obtaining an adhesive agent layer having good curability and good flexibility of the hydroxyl-terminated urethane prepolymer.

Furthermore, as the diisocyanate compound, a bi-functional isocyanate-terminated urethane prepolymer obtained by previously reacting the diisocyanate compound as described above with diol (e.g., ethylene glycol, propylene glycol, 1,4-butanediol, 1,5-pentanediol, 3-methyl-1,5-pentanediol, and 1,6-hexanediol) can be used.

### <Tin-Free Catalyst>

The tin-free catalyst is an urethanization catalyst that does not contain tin. In the present embodiment, it is preferable that any tin-containing catalyst is not used as the urethanization catalyst.

It is preferable that the adhesive agent of the present embodiment not contain tin and a tin compound from the viewpoint of obtaining an adhesive agent being low irritation and skin friendly.

Examples of the tin-free catalyst include tertiary amine catalysts, and organometallic catalysts containing metals other than tin. One catalyst may be used alone, or two or more may be used in combination. The tin-free catalyst is preferably an organometallic catalyst from the viewpoint of better reaction promoting effects.

Examples of metals contained in the organometallic catalyst containing metals other than tin include zinc, bismuth, titanium, lead, iron, cobalt, and zirconium. Among these, zinc and bismuth are preferable, and zinc is more preferable from the viewpoint of low irritation to the skin, ease of handling, and the like.

Examples of the tertiary amine catalyst include triethylamine, triethylenediamine, and 1,8-diazabicyclo[5.4.0]-7-undecene (DBU).

Examples of the organometallic catalyst containing metals other than tin include those containing zinc, bismuth, titanium, lead, iron, cobalt, and zirconium, and the organometallic catalysts containing bismuth or zinc are preferable. Examples of the organometallic catalysts containing zinc, bismuth, titanium, lead, iron, cobalt, and zirconium include zinc compounds such as zinc carboxylates, for example, zinc naphthenate and zinc 2-ethylhexanoate, and zinc acetylacetonate; bismuth compounds such as bismuth 2-ethylhexanoate and bismuth neodecanoate; titanium compounds such as dibutyltitanium dichloride, tetrabutyltitanate, and butoxytitanium trichloride; lead compounds such as lead oleate, lead 2-ethylhexanoate, lead benzoate, and lead naphthenate; iron compounds such as iron 2-ethylhexanoate and iron acetylacetonate; cobalt compounds such as cobalt benzoate and cobalt 2-ethylhexanoate; and zirconium compounds such as zirconium naphthenate, and preferable is bismuth neodecanoate or zinc carboxylate.

The amount of the tin-free catalyst used in the reaction of an oxyalkylene polymer and a diisocyanate compound is preferably more than 0.001 parts by mass and 0.1 parts by mass or less, more preferably 0.005 or more and less than 0.05 parts by mass, and further preferably 0.01 to 0.04 parts by mass per 100 parts by mass of the oxyalkylene polymer, from the viewpoint of good reaction promoting effects and reduction of amount of residues of metal components.

### (Tackifier Resin)

The tackifier resin according to the present embodiment is not particularly limited, and it may contain one or both of a tackifier resin having a softening point of 70°C or higher and a tackifier resin having a softening point of less than 70°C. It is preferable to contain a tackifier resin having a softening point of 70°C or higher, and it is also preferable to be a tackifier resin having a softening point of 70°C or higher. By containing a tackifier resin having a softening point of 70°C or higher, it is easy to obtain an adhesive agent having a sufficient adhesive force and being suppressed so as not to remain on the skin when peeled off from the skin.

Examples of the tackifier resin include rosin-based tackifier resins, terpene-based tackifier resins, alicyclic saturated hydrocarbon-based tackifier resins, styrene-based tackifier resins, and acrylic-based tackifier resins; and preferable are terpene-based tackifier resins or styrene-based tackifier resins from the viewpoint of improving the adhesive force to the skin or SUS steel plates. Terpene-based tackifier resins are more preferable from the viewpoint of good adhesive force to phenol resins. Also, styrene-based tackifier resins are more preferable from the viewpoint of less coloring of the adhesive agent.

In the adhesive composition according to the present embodiment, any of a tackifier resin having a hydroxyl group and a tackifier resin having no hydroxyl groups can be suitably contained, and a tackifier resin having no hydroxyl groups can be suitably used from the viewpoint of appropriately suppressing the adhesive force to the skin and the viewpoint of further improving the water resistance. The adhesive composition according to the present embodiment may not contain a tackifier resin having no hydroxyl groups.

Specific examples of the rosin-based tackifier resin include unmodified rosins (raw rosins) such as gum rosins, wood rosins, and tall oil rosins; modified rosins obtained by modifying these unmodified rosins through hydrogenation, disproportionation, polymerization, or the like (e.g., hydrogenated rosins, disproportionated rosins, polymerized rosins, or other chemically modified rosins; hereinafter, the same applies); and various other rosin derivatives. Examples of the rosin derivative include rosin esters such as unmodified rosins esterified with alcohol (i.e., esterified products of rosin) and modified rosins esterified with alcohol (i.e., esterified products of modified rosin); unsaturated fatty acid modified rosins obtained by modifying unmodified rosins or modified rosin with unsaturated fatty acid; unsaturated fatty acid modified rosin esters obtained by modifying rosin esters with unsaturated fatty acid; rosin alcohols obtained by reducing the carboxy groups in unmodified rosins, modified rosins, unsaturated fatty acid modified rosins, or unsaturated fatty acid modified rosin esters; metal salts of rosins (in particular, rosin esters) such as unmodified rosins, modified rosins, and various rosin derivatives; and rosin phenol resins obtained by adding phenol to rosins (e.g., unmodified rosins, modified rosins, or various rosin derivatives) with an acid catalyst and thermally polymerizing the resultant, and preferable are rosin ester-based tackifier resins.

The rosin-based tackifier resin may be a commercially available product for use. Examples thereof include "HARIESTER TF", "HARIESTER S", "NEOTALL G2", "NEOTALL 101N", "NEOTALL 125HK", "HARITACK 8LJA", "HARITACK ER95", "HARITACK SE10", "HARITACK PH", "HARITACK F85", "HARITACK F105", "HARITACK FK100", "HARITACK FK125", and "HARITACK PCJ" manufacture by Harima Chemicals, Inc.; "Foral 105-E", "Foral 85-E", and "Foral AX-E" manufactured by Eastman Chemical Company; "SUPER ESTER A-75", "SUPER ESTER A-100", "SUPER ESTER A-115", "SUPER ESTER A-125", "PENSEL A", "PENSEL AZ", "PENSEL C", "PENSEL D-125", "PINECRYSTAL KE-100", "PINECRYSTAL KE-311", "PINECRYSTAL KE-359", "PINECRYSTAL KE-604", and "PINECRYSTAL KR-140" manufactured by Arakawa Chemical Industries Ltd.; "KF382S", "KF392S", "KF364", "KF384S", "KF394S", "KF398S", "KF399S", "KF452S", "KF462S", "KF454S", "KF464S", "KP120", "KP130", "KP140", "KP150", "K107", and "K108" manufactured by GUANGDONG KOMO Co., Ltd.

Examples of the terpene-based tackifier resin include terpene resins such as α-pinene polymers, β-pinene polymers, and dipentene polymers; and modified terpene resins obtained by modifying these terpene resins (e.g., phenol modification, aromatic modification, hydrogenation modification, and hydrocarbon modification). Examples of the modified terpene resin include terpene-modified phenol resins, aromatic modified terpene resins (e.g., styrene-modified terpene resin), and hydrogenated terpene resins. Among these, aromatic modified terpene resins are preferably used. Any one or two or more of these terpene-based tackifier resins (e.g., aromatic modified terpene resins) which are different in type, characteristics (e.g., softening point), and the like, may be used in combination.

The terpene-based tackifier resin may be a commercially available product for use. Examples thereof include YS RESIN TO125 (manufactured by YASUHARA CHEMICAL CO., LTD.), YS RESIN TH130 (manufactured by YASUHARA CHEMICAL CO., LTD.), and ARKON M115 (manufactured by Arakawa Chemical Industries Ltd.).

Examples of the hydrocarbon-based tackifier resin include various hydrocarbon-based resins such as aliphatic-based (C5-based) petroleum resins, aromatic-based (C9-based) petroleum resins, aliphatic/aromatic copolymer-based (C5/C9-based) petroleum resins, hydrogenated products thereof (e.g., alicyclic-based petroleum resin (alicyclic saturated hydrocarbon resin) obtained by hydrogenating aromatic-based petroleum resin), various modified products thereof (e.g., maleic anhydride-modified product), coumarone-based resins, coumarone indene-based resins, and preferable is an alicyclic saturated hydrocarbon resin. One hydrocarbon-based tackifier resin may be used alone, or two or more may be used in combination.

Examples of the styrene-based tackifier resin include styrene homopolymers, α-methylstyrene homopolymers, α-methylstyrene-styrene copolymers, styrene-aliphatic copolymers, α-methylstyrene-styrene-aliphatic copolymers, C9-based petroleum resins, C5/C9-based petroleum resins, phenol-modified styrene resins, and hydrogenated products thereof. One styrene-based tackifier resin may be used alone, or two or more may be used in combination.

Among these, styrene homopolymers, α-methylstyrene homopolymers, α-methylstyrene-styrene copolymers, styrene-aliphatic polymers, α-methylstyrene-styrene-aliphatic copolymers, phenol-modified styrene resins, and partially hydrogenated products thereof are preferable, and styrene homopolymers, α-methylstyrene homopolymers, α-methylstyrene-styrene copolymers, and partially hydrogenated products thereof are more preferable. These are excellent in compatibility with (meth)acrylic-based block copolymers. In particular, styrene homopolymers are preferable.

The styrene-based tackifier resin may be a commercially available product for use. Examples thereof include "SYLVARES SA-85", "SYLVARES SA-100", "SYLVARES SA-120", "SYLVARES SA-140", and "SYLVARES 520" manufactured by Arizona Chemical Company; "Escorez ECR-213" and "Escorez ECR-807" manufactured by Exxon Mobil Corporation; "YS RESIN SX100" manufactured by YASUHARA CHEMICAL CO., LTD.; "FTR 0100", "FTR 2120", "FTR 2140", "FTR 6100", "FTR 6110", "FTR 6125", "FTR 7100", "FTR 8100", "FTR 8120", and "FMR 0150" manufactured by Mitsui Chemicals, Inc.; and "Kristalex F85", "Kristalex F100", "Kristalex F115", "Kristalex 1120", "Kristalex 3070", "Kristalex 3085", "Kristalex 3100", and "Kristalex 5140" manufactured by Eastman Chemical Company.

Examples of the acrylic-based tackifier resin include acrylic-based tackifier resins based on acrylic-based polymers (homopolymers or copolymers) obtained by using one or two or more (meth)acrylic acid alkyl esters as monomer components. Specific examples of the (meth)acrylic acid alkyl ester include (meth)acrylic acid C1-20 alkyl esters such as methyl (meth)acrylate, ethyl (meth)acrylate, propyl (meth)acrylate, isopropyl (meth)acrylate, butyl (meth)acrylate, isobutyl (meth)acrylate, s-butyl (meth)acrylate, t-butyl (meth)acrylate, pentyl (meth)acrylate, hexyl (meth)acrylate, heptyl (meth)acrylate, octyl (meth)acrylate, 2-ethylhexyl (meth)acrylate, isooctyl (meth)acrylate, nonyl (meth)acrylate, isononyl (meth)acrylate, decyl (meth)acrylate, isodecyl (meth)acrylate, undecyl (meth)acrylate, dodecyl (meth)acrylate, tridecyl (meth)acrylate, tetradecyl (meth)acrylate, pentadecyl (meth)acrylate, hexadecyl (meth)acrylate, heptadecyl (meth)acrylate, octadecyl (meth)acrylate, nonadecyl (meth)acrylate, and eicosyl (meth)acrylate. Among these, (meth)acrylic acid alkyl esters having a linear or branched alkyl group having 4 to 18 carbon atoms can be preferably used.

The acrylic-based polymers may contain units corresponding to other monomer components copolymerizable with the above (meth)acrylic acid alkyl esters, as necessary.

The amount of tackifier resin used per 100 parts by mass of the hydroxyl-terminated urethane prepolymer is not particularly limited, and is preferably 0.5 to 150 parts by mass, more preferably 1.0 to 100 parts by mass, further preferably 3.0 to 50 parts by mass, and yet further preferably 5.0 to 40 parts by mass, from the viewpoint of obtaining an adhesive agent having a sufficient adhesive force to a device and a sealant and sufficient long-term water resistance.

### (Polyisocyanate Compound)

The polyisocyanate compound is a curing agent for the hydroxyl-terminated urethane prepolymer.

The polyisocyanate compound is a compound having two or more isocyanate groups in a molecule; and one polyisocyanate compound may be used alone, or two or more may be used in combination.

The polyisocyanate compound is preferably a diisocyanate compound, from the viewpoint of ease of availability, reactivity, and the like. In addition, the polyisocyanate compound having 3 or more isocyanate groups in a molecule is preferable, from the viewpoint of good adhesive force, suppression of adhesive residues, and the like of the adhesive agent.

Specific examples of the diisocyanate compound include those similar to the specific examples of the diisocyanate compound constituting the hydroxyl-terminated urethane prepolymer, as described above.

Examples of the polyisocyanate compound having 3 or more isocyanate groups in a molecule include isocyanurate modified products, biuret modified products, allophanate modified products, and tri-functional or higher isocyanate-terminated urethane prepolymers (adducts) which are reaction products of a diisocyanate compound and polyol having 3 or more hydroxyl groups in one molecule (e.g., trimethylolpropane), all of which are derivatives of the diisocyanate compound described above.

The amount of polyisocyanate compound used is not particularly limited and is preferably 0.5 to 100 parts by mass, more preferably 1.0 to 50 parts by mass, further preferably 1.0 to 30 parts by mass, and yet further preferably 1.0 to 10 parts by mass based on a total of 100 parts by mass of the hydroxyl-terminated urethane prepolymer and the tackifier resin, from the viewpoint of obtaining an adhesive agent having a sufficient adhesive force to a device and a sealant and sufficient long-term water resistance.

### (Other Components)

The adhesive composition may contain other components other than the hydroxyl-terminated urethane prepolymer, the tackifier resin and the polyisocyanate compound. Examples of the other components include various additives such as a plasticizer, an antioxidant, an antistatic agent, a filler, an ultraviolet absorber, a light stabilizer, a conductivity imparting agent, a leveling agent, and a polyol. Also, a solvent may be contained. The other components may be used alone, or two or more may be used in combination; and these can be blended at a content within a range that does not impair the effects of the present invention.

The total content of the hydroxyl-terminated urethane prepolymer, the tackifier resin and the polyisocyanate compound in the adhesive composition (excluding the solvent) is preferably 85% by mass or more, more preferably 90% by mass or more, further preferably 95% by mass or more, and yet further preferably 100% by mass from the viewpoint of sufficiently exerting the effects of the present invention.

The plasticizer can be selected from the viewpoint of compatibility with other components contained, wettability of the adhesive agent layer to an adherend, and the like, and examples thereof include fatty acid ester and phosphoric acid ester, that have 8 to 30 carbon atoms.

The antioxidant contributes to the suppression of thermal deterioration of the adhesive agent layer, or the like; examples thereof include phenol-based, amine-based, sulfur-based, and phosphorous-based antioxidants, and among these, the phenol-based antioxidant is preferable from the viewpoint of low irritation to the skin.

The antistatic agent contributes to the suppression of damage to the circuit pattern due to electrostatic discharge, and examples thereof include inorganic salts; ionic liquids containing imidazolium ions, pyridinium ions, ammonium ions, or the like; and surfactants.

Examples of the filler include talc, calcium carbonate, and titanium oxide.

Examples of the ultraviolet absorber include benzophenone-based, benzotriazole-based, salicylic acid-based, oxalic acid anilide-based, cyanoacrylate-based, and triazine-based ultraviolet absorbers.

Examples of the light stabilizer include hindered amine-based light stabilizers and ultraviolet stabilizers.

Examples of the conductivity imparting agent include metal microparticles such as silver nanoparticles.

Examples of the leveling agent include polymer-based leveling agents such as acrylic-based, vinyl-based, silicone-based, and fluorine-based leveling agent.

The adhesive composition of the present embodiment may or may not further contain polyols as the other components. The number-average molecular weight of the polyol is preferably 5,000 or less, and more preferably 600 to 3,000, from the viewpoint of improving the adhesive force of the adhesive agent.

However, according to the adhesive composition of the present embodiment, it is easy to obtain an adhesive agent having a sufficient adhesive force even though a polyol having a number-average molecular weight of 5,000 or less is not contained. The content of polyol having a number-average molecular weight of 5,000 or less in the adhesive composition is preferably less than 2.5 parts by mass, more preferably 0 to 2.0 parts by mass, and further preferably 0 to 1.0 parts by mass, per 100 parts by mass of the hydroxyl-terminated urethane prepolymer, from the viewpoint of suppressing damage to the skin due to an excessively increased adhesive force.

Similarly, the content of polyol other than the hydroxyl-terminated urethane prepolymer in the adhesive composition is preferably less than 2.5 parts by mass, more preferably 0 to 2.0 parts by mass, and further preferably 0 to 1.0 parts by mass, per 100 parts by mass of the hydroxyl-terminated urethane prepolymer.

### [Method for Producing Adhesive Composition]

The method for producing an adhesive composition of the present embodiment is a method for producing an adhesive composition by reacting an oxyalkylene polymer having a number-average molecular weight of 3,000 or more and a diisocyanate compound to obtain a hydroxyl-terminated urethane prepolymer, and mixing the hydroxyl-terminated urethane prepolymer, a tackifier resin, and a polyisocyanate compound.

The hydroxyl-terminated prepolymer thus obtained is reacted with the polyisocyanate compound to suitably obtain the adhesive composition of the present embodiment described above.

Note that, when the hydroxyl-terminated urethane prepolymer, the tackifier resin, and the polyisocyanate compound are mixed, the other components that can be contained in the adhesive composition described above may also be mixed.

The hydroxyl-terminated urethane prepolymer can be preferably obtained by reacting an oxyalkylene polymer having a number-average molecular weight of 3,000 or more and a diisocyanate compound in the presence of a tin-free catalyst. The oxyalkylene polymer having a number-average molecular weight of 3,000 or more, the diisocyanate compound, and the tin-free catalyst are the same as those described above.

The hydroxyl-terminated urethane prepolymer can be produced, for example, by a method in which the oxyalkylene polymer, the diisocyanate compound, the tackifier resin, a catalyst and a solvent are all charged at once in a reaction vessel, or a method in which the diisocyanate compound is subsequently added dropwise or the like in a reaction vessel where the oxyalkylene polymer, the tackifier resin, a catalyst and a solvent have been charged.

Examples of the solvent include ketons such as acetone, and methylethylketon; esters such as ethyl acetate; and aromatic hydrocarbons such as toluene, and xylene. One solvent may be used alone, or two or more may be used in combination.

When using a solvent, the amount thereof used is preferably 50 to 500 parts by mass, more preferably 70 to 400 parts by mass, and further preferably 80 to 300 parts by mass, based on a total of 100 parts by mass of the oxyalkylene polymer, from the viewpoint of uniformity of the reaction systems and synthesis efficiency.

It is preferable that the oxyalkylene polymer and the diisocyanate compound be reacted at the isocyanate index of 100 or less, preferably 30 to 95, and more preferably 50 to 95, from the viewpoint of efficiently obtaining a hydroxyl-terminated urethane prepolymer having an appropriate molecular chain length.

The reaction temperature is preferably less than 100°C, more preferably 70 to 95°C, and further preferably 75 to 90°C, from the viewpoint of promoting the urethanization reaction and suppressing the side reaction.

After completion of the reaction, a terminator for reaction such as acetylacetone may be added thereto in order to quench the catalyst.

The average number of hydroxyl groups per molecule of the hydroxyl-terminated urethane prepolymer is preferably 3.0 or less, more preferably 1.7 to 2.9, and further preferably 1.8 to 2.5, from the viewpoint of obtaining an adhesive agent having a good balance between the adhesive force to the skin and the adhesive force to a device.

The isocyanate index of the polyisocyanate compound used to react with the hydroxyl-terminated urethane prepolymer is preferably more than 100, more preferably 101 to 1,500, and further preferably 105 to 1,000.

### [Adhesive Agent]

The adhesive agent of the present embodiment is a cured product of the adhesive composition of the present embodiment described above.

An adhesive agent can be obtained by reacting and curing the hydroxyl-terminated urethane prepolymer and the polyisocyanate compound in the adhesive composition.

As described later, an adhesive agent can be formed as an adhesive agent layer by, for example, applying and curing the adhesive composition on a substrate.

For the reaction of the hydroxyl-terminated urethane prepolymer and the polyisocyanate compound, a catalyst and a solvent can be used as necessary. As the solvent, those described in the above [Method for Producing Adhesive Composition] can be suitably used, and these solvents may be the same or different, but are preferably the same.

When using a catalyst, a urethanization catalyst, the tin-free catalyst described above, can be used. The catalyst may be same as or different from those used in the synthesis of the hydroxyl-terminated urethane prepolymer. When the catalyst used in the synthesis of the hydroxyl-terminated urethane prepolymer remains, the remaining catalyst can also act as a catalyst in the reaction of the hydroxyl-terminated urethane prepolymer and the polyisocyanate compound.

When the catalyst is added in the reaction of the hydroxyl-terminated urethane prepolymer and the polyisocyanate compound, the amount to be added is more than 0.001 parts by mass and 0.1 parts by mass or less, preferably 0.005 to 0.05 parts by mass, and more preferably 0.01 to 0.04 parts by mass, based on a total of 100 parts by mass of the hydroxyl-terminated urethane prepolymer and the polyisocyanate compound. When the catalyst is added, after completion of the reaction, it is preferable to add a reaction terminator in order to quench the catalyst.

Examples of the solvent used in the reaction of the hydroxyl-terminated urethane prepolymer and the polyisocyanate compound include those similar to the case of the above-mentioned hydroxyl-terminated urethane prepolymer. The catalyst may be same as or different from those used in the synthesis of the hydroxyl-terminated urethane prepolymer. When the solvent used in the synthesis of the hydroxyl-terminated urethane prepolymer remains, it can be used as it is.

When using a solvent, the amount thereof used is preferably 50 to 500 parts by mass, more preferably 70 to 400 parts by mass, and further preferably 80 to 300 parts by mass, based on a total of 100 parts by mass of the hydroxyl-terminated urethane prepolymer and the polyisocyanate compound.

The reaction temperature between the hydroxyl-terminated urethane prepolymer and the polyisocyanate compound is preferably less than 120°C, more preferably 40 to 110°C, and further preferably 50 to 100°C, from the viewpoint of promoting the urethanization reaction and suppressing the side reaction.

### [Patch]

The patch of the present embodiment has a substrate and an adhesive agent layer provided on at least a part of the surface of the substrate, and the adhesive agent layer contains the above-mentioned adhesive agent of the present embodiment.

The patch having the adhesive agent layer formed by using the adhesive composition of the present embodiment can provide an adhesive agent having sufficient adhesion to a device and a sealant and sufficient long-term water resistance.

The form of the patch may be a desired sheet shape of a size according to the usage mode, or may be cut to a desired size for use.

The patch is preferably an adhesive tape from the viewpoint of convenience.

The adhesive composition used to form an adhesive agent layer may be a one-component type in which a hydroxyl-terminated urethane prepolymer and a polyisocyanate compound, which is a curing agent, are mixed in advance, or a two-component type consisting of a first component that contains a hydroxyl-terminated urethane prepolymer and a second component that contains a polyisocyanate compound, which is a curing agent, depending on the usage mode of the patch, or the like.

The thickness of the adhesive agent layer is preferably 5 to 100 µm, more preferably 10 to 80 µm, and further preferably 20 to 50 µm, from the viewpoint of securing good adhesive force on the skin.

The substrate is preferably made of a material that has good followability of the patch to the shapes of the skin or a device, and that is less likely to be broken when the patch is used, and examples thereof include those made of a resin film, fabric, paper, or the like. The substrate may be used alone, or two or more may be used in combination, and it may also be a composite material by laminating layers or the like.

Examples of the material for the resin film include polyurethane; polyamide; acrylic resin; polyolefins such as polyethylene, polypropylene, and ethylene-vinyl acetate copolymer; and polyester, and preferable is a polyester film.

Examples of the fabric include woven fabric, non-woven fabric, knitted fabric, and net, and the properties of the material may be a resin, which is a material for the above-mentioned resin film, and natural fibers such as cotton, silk, and wool.

The thickness of the substrate is not particularly limited as long as the followability to the shapes of the skin or the device is secured, and the substrate is not easily broken when the patch is used, and the thickness is preferably 10 to 500 µm and more preferably 20 to 400 µm.

The surface of the adhesive agent layer in the patch is preferably covered with a release liner for the protection of the surface of the adhesive agent layer before using the patch. The release liner is appropriately peeled off from the surface of the adhesive agent layer when the patch is used.

A known release liner used for adhesive tapes for skin can be applied to the release liner. Examples of the release liner include woodfree paper, glassine paper, or parchment paper on the surface of which a release agent such as a silicone resin or fluororesin is coated, and woodfree paper anchor coated or polyethylene laminated with resin, on the surface of which a release agent such as a silicone resin or fluororesin is coated. In addition, as the release liner, a resin film having transparency can be used, and examples thereof include a polyester film.

The thickness of the release liner is not particularly limited as long as it can protect the adhesive agent layer and it can be easily peeled when used, and examples thereof are 15 to 200 µm.

The adhesive agent layer may be provided, for example, on a part of the surface of the substrate made of a resin film at one or two or more locations, may be provided on only a part of one side, or may be provided on the entire surface of one side. Also, it may be provided on both sides of the surface of the substrate.

When the adhesive agent layer is adhered to a sealant of a device, or the like, for example, the adhesive force to a phenol resin, which is a common material as the sealant, is preferably 2.5 N/15 mm or more, and more preferably 3.0 N/15 mm or more, from the viewpoint of sufficiently fixing a device.

Similarly, after the adhesive agent layer is pasted to the phenol resin and immersed in a container containing tap water at 23°C for 7 days, the adhesive force to the phenol resin is preferably 1.0 N/15 mm or more, more preferably 2.5 N/15 mm or more, and further preferably 3.0 N/15 mm or more, from the viewpoint of sufficiently fixing a device.

When the adhesive agent layer is adhered to a sealant of a device, or the like, for example, the adhesive force to a SUS steel plate, which is a common material as the device, is preferably 2.0 N/15 mm or more, more preferably 2.5 N/15 mm or more, and further preferably 3.0 N/15 mm or more, from the viewpoint of sufficiently fixing the device.

Note that the adhesive force used herein is a value measured by a method in accordance with JIS Z 0237:2009, and specifically, it is measured by the methods described in Examples.

The method for producing a patch can be performed using a known method, and examples thereof include a method of applying and curing an adhesive composition on a release liner to form an adhesive agent layer, and adhering the substrate thereon. Another example is a method of applying and curing an adhesive composition on the substrate to form an adhesive agent layer, and adhering the release liner thereon.

The method of applying the adhesive composition is not particularly limited, and for example, known methods such as bar coding, knife coating, roll coating, blade coating, die coating, microgravure coating, comma coating, slot die coating, lip coating, and cast coating can be applied.

After application, adhesive composition is preferably cured by, for example, drying with hot air at 40 to 80°C to sufficiently volatilize the solvent contained in the adhesive composition, and thereby forming an adhesive agent layer.

### [Wearable Device]

The wearable device of the present embodiment has a wearable device main body and an adhesive agent layer, and the adhesive agent layer is provided on at least a part of the surface of the wearable device facing the skin and contains the adhesive agent of the present embodiment.

When the above-mentioned adhesive composition of the present embodiment is used, an adhesive agent layer can be formed that has a good balance between the adhesive force to the skin and the adhesive force to the device and is skin friendly. Therefore, the adhesive agent of the present embodiment is suitable for applying to a wearable device that is adhered to the skin.

The adhesive agent layer can be formed, for example, in the above-mentioned form of the patch.

Further, the adhesive agent layer may be formed in a manner of being sandwiched between release liners on both sides, and the wearable device main body and the skin may be adhered only via an adhesive agent layer, without using a substrate.

Examples of the wearable device pasted to the skin include a biosensor and environmental sensor. Examples of subjects detected by the biosensors include electrocardiography, pulse wave, body temperature, acceleration, heartbeat interval, pulse interval, respiration interval, electrocardiogram, electromyogram, activity level, blood pressure, and electroencephalogram.

### [Wearable Device Kit]

The wearable device kit of the present embodiment contains a wearable device main body, and at least one of the adhesive agent of the present embodiment and the patch of the present embodiment
In this way, the wearable device main body may be provided with an adhesive agent or a patch as another component, and comprised such that an adhesive agent can be formed when in use, not in the manner that the adhesive agent layer has in advice.

Either of the adhesive agent and patch may be used alone, or both may be used in combination.

### Examples

Hereinafter, the present invention will be specifically described based on Examples; however, the present invention is not limited to the following Examples, and various modifications can be made without departing from the scope of the present invention.

### [Measuring Methods]

Methods for measuring various physical properties in the following Synthesis Examples are as follows.

### <Number-Average Molecular Weight (Mn) and Weight-Average Molecular Weight (Mw)>

Mn and Mw were measured with gel permeation chromatography (GPC) in the following measurement conditions (in terms of polystyrene), the molecular weight distribution (Mw/Mn) was calculated from these values.

### [Measurement Conditions]

- Apparatus used: "HLC-8320GPC", manufactured by Tosoh Corporation
- Column used: "TSKgel (registered trademark) SuperMultiporeHZ-M", manufactured by Tosoh Corporation
- Detector: Differential refractive index (RI) detector
- Detected temperature: 40°C
- Eluent: Tetrahydrofuran
- Flow rate: 0.350 mL/min
- Sample concentration: 0.5% by mass
- Amount of sample infused: 10 µL
- Standard sample: Polystyrene

### <Content of Ethylene Oxide (EO) Units>

The content of EO units of each oxyalkylene polymer was based on the amount of propylene oxide (PO) and EO charged used for the synthesis, and the amount of EO charged was considered as the content of EO units in the oxyalkylene polymer.

The content of EO units of the hydroxyl-terminated urethane prepolymer was designated as the weight-averaged value of the content of EO units in each component of the raw material.

### <Hydroxyl Value>

The hydroxyl value was determined in accordance with B method (automatic potentiometric titration method) of JIS K 1557-1:2007.

### <Degree of Unsaturation>

The degree of unsaturation was measured in accordance with JIS K 1557-3:2007.

### <Average Number of Hydroxyl Groups Per Molecule of Oxyalkylene Polymer A>

The number of hydroxyl groups per molecule of each of the oxyalkylene polymers A1 and A2 used as the oxyalkylene polymer A (the number of hydroxyl groups per molecule of the initiator used for the synthesis of each oxyalkylene polymer) was weight-averaged based on the amount of each oxyalkylene polymer blended, and the obtained value was considered as the average number of hydroxyl groups per molecule of the oxyalkylene polymer A.

### <Average Number of Hydroxyl Groups Per Molecule of Hydroxyl-Terminated Urethane Prepolymer>

The number of hydroxyl groups per molecule of each of the oxyalkylene polymers and each of the diisocyanate compounds used for the synthesis of the hydroxyl-terminated urethane prepolymer was weight-averaged based on each of the amounts of the oxyalkylene polymers and each of the amounts of the diisocyanate compounds blended, and the obtained value was considered as the average number of hydroxyl groups per molecule.

### [Substances Used]

Substances used in the following Synthesis Examples and Examples are shown below in detail.
(1) Initiator
   - TBA-DMC catalyst: zinc hexacyanocobaltate complex with tert-butanol as the ligand.
(2) Absorbent
   - Absorbent: Synthesized magnesium silicate; "KYOWAAD (registered trademark) 600S", manufactured by Kyowa Chemical Industry Co., Ltd.
(3) Polyisocyanate Compound
   - 50M-HDI: Hexamethylene diisocyanate (HDI); "Duranate (registered trademark) 50M-HDI", manufactured by Asahi Kasei Corporation
   - D201: HDI-based bifunctional isocyanate-terminated prepolymer; "Duranate (registered trademark) D201", manufactured by Asahi Kasei Corporation
(4) Urethanization Catalyst
   - Bismuth neodecanoate; bismuth (Bi)-containing urethanization catalyst; manufactured by Sigma Aldrich CO. LLC
   - XK627: zinc carboxylate; zinc (Zn)-containing urethanization catalyst; "K-KAT XK627", manufactured by KING INDUSTRIES, Inc.
(5) Curing Agent
   - Curing agent (polyisocyanate compound): HDI-based polyisocyanate; "Duranate (registered trademark) E402-80B", manufactured by Asahi Kasei Corporation; Solid content of 80% by mass
(6) Tackifier Resin
   - Styrene resin: "YS RESIN (registered trademark) SX100", manufactured by YASUHARA CHEMICAL CO., LTD., softening point of 100°C
   - Terpene phenol resin: "YS RESIN (registered trademark) TH130", manufactured by YASUHARA CHEMICAL CO., LTD., softening point of 130°C
   - Alpha methylstyrene resin: "SYLVARES SA-140", manufactured by Kraton Polymer Japan Ltd., softening point of 137°C
   - Phenol-modified alpha-methylstyrene resin: "SYLVARES 520", manufactured by Kraton Polymer Japan Ltd., softening point of 75°C
(7) Oxyalkylene Polymer
   - A1: oxyalkylene polymer synthesized in Synthesis Example 1-1
   - A2: oxyalkylene polymer synthesized in Synthesis Example 1-2
   - B1: oxyalkylene polymer synthesized in Synthesis Example 1-3
   - A3: oxyalkylene polymer synthesized in Synthesis Example 1-4
   - A'1: "EXCENOL 903", manufactured by AGC Inc.
   - A'2: "EXCENOL 2026T", manufactured by AGC Inc.
   - B'1: "PREMINOL 1002", manufactured by AGC Inc.

### [Synthesis of Oxyalkylene Polymer]

### (Synthesis Example 1-1)

In a pressure-proof container, as the initiator, 1000 g of glycerin and TBA-DMC catalyst (metal concentration of 46 ppm by mass) was charged, and after nitrogen substitution in the container, the reaction solution was heated to 135°C while being stirred to react by adding 120g of propylene oxide (PO).

The reaction solution was cooled to 135°C after the temperature increase stopped, 3782.4 g of PO and 945.6 g of ethylene oxide (EO) were added into the container while stirring the reaction solution, and then after confirming that there was no more change in inner pressure, the absorbent was added thereto and neutralization and catalyst removal were performed to obtain oxyalkylene polymer A1.

### (Synthesis Example 1-2)

Oxyalkylene polymer A2 was synthesized in the same manner as in Synthesis Example 1-1, except that the initiator in Synthesis Example 1-1 was changed from glycerin to propylene glycol.

### (Synthesis Example 1-3)

Oxyalkylene polymer B1 was synthesized in the same manner as in Synthesis Example 1-1, except that the initiator in Synthesis Example 1-1 was changed from glycerin to n-butanol, after the reaction solution was cooled, no EO was added but 2364 g of PO was added.

### (Synthesis Example 1-4)

Oxyalkylene polymer A3 was synthesized in the same manner as in Synthesis Example 1-1, except that the initiator in Synthesis Example 1-1 was changed from glycerin to propylene glycol, and after the reaction solution was cooled, no EO was added.

Various physical properties of the oxyalkylene polymers A1 to A3 and B1 synthesized as described above, as well as the commercially available oxyalkylene polymers A'1, A'2, and B'1 are shown in Table 1.

### [Table 1]

**Table 1**

| Oxyalkylene polymer | | Number of hydroxyl groups | Number-average molecular weight | Molecular weight distribution | Degree of unsaturation | Hydroxyl value | Content of EO units |
|---|---|---|---|---|---|---|---|
| | | (-) | (Mn) | (Mw/Mn) | [meq/g] | [mgKOH/g] | (% by mass) |
| Synthesis example 1-1 | A1 | 3 | 8,500 | 1.19 | 0.013 | 17.6 | 20 |
| Synthesis example 1-2 | A2 | 2 | 9,500 | 1.06 | 0.011 | 11.8 | 20 |
| Synthesis example 1-3 | B1 | 1 | 5,300 | 1.09 | 0.007 | 11.5 | 0 |
| Synthesis example 1-4 | A3 | 2 | 9,700 | 1.17 | 0.012 | 11.6 | 0 |
| Commercially available product | A'1 | 3 | 1,500 | - | - | - | 20 |
| | A'2 | 2 | 2,200 | - | - | - | 14.5 |
| | B'1 | 1 | 2,000 | - | - | - | 0 |

### [Synthesis of Hydroxyl-Terminated Urethane Prepolymer]

### (Synthesis Example 2-1)

In a reaction vessel equipped with a thermometer, a stirrer, and a cooling tube, 45 parts by mass of oxyalkylene polymer A1, 35 parts by mass of oxyalkylene polymer A3, 20 parts by mass of oxyalkylene polymer B1, 50.6 parts by mass of toluene and 50.6 parts by mass of ethyl acetate were charged, 0.038 parts by mass of K-KAT XK627 was added, and the mixture was mixed at 40°C, and then 1.7 parts by mass of HDI (isocyanate index of 85) was added thereto to react at 80°C.

While appropriately diluting the mixture with ethyl acetate, the mixture was maintained at 80°C and reacted for 7 hours to obtain a solution of transparent hydroxyl-terminated urethane prepolymer at a concentration of 50% by mass (urethane prepolymer U1; the average number of hydroxyl groups of 2.2, the content of EO units of 9% by mass).

### (Synthesis Example 2-2)

As shown in Table 2, a solution of hydroxyl-terminated urethane prepolymer at a concentration of 50% by mass (also referred to as "urethane prepolymer U2") was prepared in the same manner as in Synthesis Example 2-1, except that the diisocyanate compound HDI was changed to D-201 (isocyanate index of 85), and its amount added was changed.

### (Synthesis Example 2-3)

As shown in Table 2, a solution of hydroxyl-terminated urethane prepolymer at a concentration of 50% by mass (also referred to as "urethane prepolymer U3") was prepared in the same manner as in Synthesis Example 2-1, except that the catalyst K-KAT XK-627 was changed to bismuth neodecanoate, and its amount added was changed.

### (Synthesis Example 2-4)

As shown in Table 2, a solution of hydroxyl-terminated urethane prepolymer at a concentration of 50% by mass (also referred to as "urethane prepolymer U4") was prepared in the same manner as in Synthesis Example 2-2, except that the catalyst K-KAT XK-627 was changed to bismuth neodecanoate, and its amount added was changed.

### (Synthesis Example 2-5)

A solution of hydroxyl-terminated urethane prepolymer at a concentration of 50% by mass (also referred to as "urethane prepolymer U5") was prepared in the same manner as in Synthesis Example 2-1, except that the type and amount of the oxyalkylene polymer blended were changed as shown in Table 2.

### (Synthesis Example 2-6)

A solution of hydroxyl-terminated urethane prepolymer at a concentration of 50% by mass (also referred to as "urethane prepolymer U6") was prepared in the same manner as in Synthesis Example 2-1, except that the type and amount of the oxyalkylene polymer blended were changed as shown in Table 2.

The blending compositions of the oxyalkylene polymers, diisocyanate compounds, and catalysts in Synthesis Examples 2-1 to 2-6, and the like, are shown in Table 2.

### [Table 2]

**Table 2**

| Synthesis example | | | 2-1 | 2-2 | 2-3 | 2-4 | 2-5 | 2-6 |
|---|---|---|---|---|---|---|---|---|
| Hydroxyl-terminated urethane prepolymer | | | U1 | U2 | U3 | U4 | U5 | U6 |
| Amount blended [part by mass] | Oxyalkylene polymer | A1 | 45 | 45 | 45 | 45 | 36 | - |
| | | A2 | - | - | - | - | 48 | - |
| | | B1 | 20 | 20 | 20 | 20 | 16 | - |
| | | A3 | 35 | 35 | 35 | 35 | - | - |
| | | A'1 | - | - | - | - | - | 36 |
| | | A'2 | - | - | - | - | - | 48 |
| | | B'1 | - | - | - | - | - | 16 |
| | Diisocyanate compound | HDI | 1.7 | - | 1.7 | - | 1.7 | 1.7 |
| | | D-201 | - | 5.6 | - | 5.6 | - | - |
| | Catalyst | K-KAT XK-627 | 0.038 | 0.038 | - | - | 0.038 | 0.038 |
| | | Bismuth neodecanoate | - | - | 0.013 | 0.013 | - | - |
| Physical properti es, etc. | Diisocyanate compound | Isocyanate index | 85 | 85 | 85 | 85 | 85 | 85 |
| | Oxyalkylene polymer A | Content of EO units [% by mass] | 11 | 11 | 11 | 11 | 20 | 17 |
| | | Average number of hydroxyl groups per molecule [-] | 2.6 | 2.6 | 2.6 | 2.6 | 2.4 | 2.4 |
| | Oxyalkylene polymer | Average number of hydroxyl groups per molecule [-] | 2.3 | 2.3 | 2.3 | 2.3 | 2.2 | 2.2 |
| | | Number-average molecular weight (Mn) | 8,300 | 8,300 | 8,300 | 8,300 | 8,500 | 1,900 |
| | | Content of EO units [% by mass] | 9 | 9 | 9 | 9 | 17 | 14 |
| | Hydroxyl-terminated urethane prepolymer | Average number of hydroxyl groups per molecule [-] | 2.2 | 2.1 | 2.2 | 2.1 | 2.2 | 2.2 |
| | | Content of EO units [% by mass] | 9 | 9 | 9 | 9 | 17 | 14 |
| | | Weight-average molecular weight (Mw) | 292,0 00 | 265,0 00 | 253,0 00 | 276,0 00 | 252,0 00 | 123,0 00 |

### [Production of Patch]

### (Example 1)

180 Parts by mass of a 50% by mass solution of hydroxyl-terminated urethane prepolymer U1 (90 parts by mass in terms of hydroxyl-terminated urethane prepolymer U1), 10 parts by mass of tackifier resin YS RESIN SX100, and 5 parts by mass of a curing agent were mixed to obtain an adhesive composition. After the adhesive composition was deaerated, the resulting product was coated on a polyester film (thickness of 38 µm) as the release liner with a knife coater and dried at 100°C for 2 minutes to form an adhesive agent (adhesive agent layer) of 25 µm thickness. On the adhesive agent layer, a polyester film (thickness of 75 µm) was adhered as a substrate and cured in a hot air dryer at 40°C for 3 days to produce a patch.

### (Examples 2 to 14)

Patches were produced in the same manner as in example 1, except that the blending compositions were changed as shown in Table 3.

### (Example 15)

A patch was produced in the same manner as in example 1, except that the substrate was changed from a polyester film (thickness of 75 µm) to a non-woven fabric (thickness of 200 µm).

### (Examples 16 to 28)

Patches were produced in the same manner as in examples 2 to 14, except that the substrate was changed from a polyester film (thickness of 75 µm) to a non-woven fabric (thickness of 200 µm).

### [Evaluation of Patch]

The following various evaluations were performed for each of the patches produced in the above examples 1 to 28. The evaluation results for examples 1 to 14 are shown in Table 3. Examples 1 to 10 are Examples, and examples 11 to 14 are Comparative Examples.

The similar evaluation results were also obtained for examples 15 to 28, in which the substrate was changed from a polyester film (thickness of 75 µm) to a non-woven fabric (thickness of 200 µm). Examples 15 to 24 are Examples, and examples 25 to 28 are Comparative Examples.

### <Adhesive Force>

### (Versus Phenol Resin (Left Still for 20 Minutes))

The patch (100 mm high and 15 mm wide) from which the release liner was peeled off was overlapped on the side of the adhesive agent layer with a phenol resin plate ("Sumilite (registered trademark) PL-1102", manufactured by Sumitomo Bakelite Co., Ltd.; 125 mm high, 30 mm wide, 2 mm thick), and a rubber roller was rolled over thereon from the side of the substrate of the patch with a load of 2 kg and a rate of 300 mm/minute for one return to compression bond the adhesive agent layer to the phenol resin plate.

After leaving still for 20 minutes, the adhesive force when the adherend was a phenol resin (versus phenol resin) was measured by peeling off the patch at a peeling angle of 90° and a rate of 300 mm/minute by a method in accordance with JIS Z 0237:2009.

Table 3 shows the average value of three measurement values. Also, the measured adhesive force was evaluated based on the following evaluation criteria.

### [Evaluation Criteria (Versus Phenol Resin)]

A: 3.0 N/15 mm or more
B: 2.5 N/15 mm or more and less than 3.0 N/15 mm
C: 2.0 N/15 mm or more and less than 2.5 N/15 mm
D: less than 2.0 N/15 mm

When evaluated as evaluation A, it can be said that the adhesive force to the phenol resin is sufficient, which is a representative material for the sealant of the circuit pattern for a wearable device, or the like. On the other hand, when evaluated as evaluations B to D, the adhesive force to the phenol resin was insufficient.

### (Versus Phenol Resin (23°C 100% After 7 Days))

The adhesive agent layer was compression bonded to the phenol resin plate in the same manner as in the above (Versus Phenol Resin (Left Still for 20 Minutes)).

The phenol resin plate to which the adhesive agent layer had been compression bonded was immersed in a container containing tap water at 23°C.

After 7 days, the phenol resin plate was removed from the container and lightly wiped to remove moisture, and then the adhesive force was measured within 30 minutes by peeling off the adhesive agent layer at a peeling angle of 90° and a rate of 300 mm/minute.

Table 3 shows the average value of three measurement values. Also, the measured adhesive force was evaluated based on the following evaluation criteria.

In this test, the adhesive agents having evaluation B or higher are excellent in long-term water resistance and can be used without problems in practical use. When evaluated as evaluation A, the adhesive agent is particularly excellent in long-term water resistance.

### [Evaluation Criteria (Phenol Resin Plate 23°C 100% After 7 Days)]

A: 2.0 N/15 mm or more
B: 1.0 N/15 mm or more and less than 2.0 N/15 mm
C: less than 1.0 N/15 mm

### (Versus SUS)

Each patch (100 mm high and 25 mm wide) from which the release liner was peeled off was overlapped on the side of the adhesive agent layer with a SUS steel plate (manufactured by Standard Test Piece K.K.; 125 mm high, 30 mm wide, 2 mm thick), and a rubber roller was rolled over thereon from the side of the substrate of the patch with a load of 2 kg and a rate of 600 mm/minute for one return to compression bond the adhesive agent layer to the SUS steel plate.

After leaving still for 20 minutes, the adhesive force was measured by peeling off the patch at a peeling angle of 90° and a rate of 300 mm/minute by a method in accordance with JIS Z 0237:2009.

Table 3 shows the average value of three measurement values. Also, the measured adhesive force was evaluated based on the following evaluation criteria.

### [Evaluation Criteria (SUS)]

A: 3.0 N/15 mm or more
B: 2.5 N/15 mm or more and less than 3.0 N/15 mm
C: less than 2.5 N/15 mm

When evaluated as evaluation A and B, it can be said that the adhesive force to the SUS steel plate is sufficient, which is the standard of JIS Z 0237:2009.

### (Versus Skin)

The adhesive force was measured for the case where the adherend was the skin of the forearm of humans (3 subjects) (versus skin), in the same manner as in the case where the adherend was the phenol resin.

Table 3 shows the average value of measurement values. Also, the measured adhesive force was evaluated based on the following evaluation criteria.

### [Evaluation Criteria (Versus Skin)]

A: 1.0 N/15 mm or more and 3.0 N/15 mm or less
B: more than 3.0 N/15 mm and 5.0 N/15 mm or less
C: less than 1.0 N/15 mm
D: more than 5.0 N/15 mm

When evaluated as evaluation A, it can be said that the adhesive force is adequate for the skin. When evaluated as evaluation B, the adhesive force is sufficient but slightly strong, and it may cause resistance when peeling off, compared to the case of evaluation A. When evaluated as evaluation C, the adhesive force is too weak, and it is peeled off easily. When evaluated as evaluation D, the adhesive force is too strong, and it is difficult to peel off. When evaluated as evaluation A and B, it has an adhesive force adequate for practical use.

### <Skin Observation>

A specimen (20 mm high and 15 mm wide) of the patch was attached to the skin of forearm of humans (3 subjects) in an atmosphere of 23°C and 50% RH. After one hour, the specimen was peeled off from the skin, and the skin condition was visually observed.

The observation results were evaluated based on the following evaluation criteria.

In this test, the adhesive agents having evaluation B or higher are safe for applying to the skin and can be used without problems in practical use. The adhesive agents having evaluation A are particularly safe for applying to the skin.

### [Evaluation Criteria]

A: No change from before the specimen being attached.
B: Some subjects had redness on the skin.
C: Some subjects had adhesive residues (residues of adhesive agent).

### [Table 3]

**Table 3**

| | | | | Example 1 | Example 2 | Example 3 | Example 4 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|---|---|
| Adhesive Composition [part by mass] | Hydroxyl-terminated urethane prepolymer | U1 | | 90 | 90 | 90 | 90 | - | - | - | - | 75 | 75 | 100 | - | - | - |
| | | U2 | | - | - | - | - | 90 | - | - | - | - | - | - | 100 | - | - |
| | | U3 | | - | - | - | - | - | 90 | - | - | - | - | - | - | - | - |
| | | U4 | | - | - | - | - | - | - | 90 | - | - | - | - | - | - | - |
| | | U5 | | - | - | - | - | - | - | - | 90 | - | - | - | - | - | - |
| | | U6 | | - | - | - | - | - | - | - | - | - | - | - | - | 100 | 100 |
| | Curing agent | Duranate E402-80B | | 5 | 5 | 5 | 5 | 5 | 5 | 5 | 5 | 5 | 5 | 5 | 5 | 5 | 5 |
| | Tackifier resin | YS RESIN SX100 | | 10 | - | - | - | 10 | 10 | 10 | 10 | - | - | - | - | - | 10 |
| | | YS POLYSTER TH130 | | - | 10 | - | - | - | - | - | - | - | - | - | - | - | - |
| | | SYLVARES SA140 | | - | - | 10 | - | - | - | - | - | 25 | - | - | - | - | - |
| | | SYLVARES 520 | | - | - | - | 10 | - | - | - | - | - | 25 | - | - | - | - |
| Film layer thickness of adhesive agent [µm] | | | | 25 | 25 | 25 | 25 | 25 | 25 | 25 | 25 | 25 | 25 | 25 | 25 | 25 | 25 |
| Characteristics | Adhesive force | Versus phenol resin | [N/15mm] | 4.0 | 5.6 | 4.8 | 4.3 | 3.8 | 4.2 | 3.8 | 6.8 | 7.6 | 7.5 | 4.4 | 6.0 | 2.1 | 3.0 |
| | | | Evaluation | A | A | A | A | A | A | A | A | A | A | A | A | C | A |
| | | Versus phenol resin 23°C 100% after 7 days | [N/15mm] | 3.0 | 3.1 | 3.5 | 2.6 | 2.1 | 2.8 | 2.2 | 1.4 | 5.1 | 4.5 | 3.7 | 1.1 | 0.1 | 0.3 |
| | | | Evaluation | A | A | A | A | A | A | A | B | A | A | A | B | C | C |
| | | Versus SUS | [N/15mm] | 2.8 | 3.8 | 4.0 | 2.9 | 2.5 | 2.6 | 2.6 | 3.6 | 4.7 | 3.9 | 1.5 | 1.2 | 0.5 | 2.3 |
| | | | Evaluation | B | A | A | B | B | B | B | A | A | A | C | C | C | C |
| | | Versus skin | [N/15mm] | 2.8 | 3.4 | 3.1 | 3.6 | 2.1 | 2.7 | 3.1 | 4.2 | 4.7 | 4.6 | 2.3 | 2.9 | 0.8 | 1.8 |
| | | | Evaluation | A | B | B | B | A | A | B | B | B | B | A | A | C | A |
| | Appearance when applied to the skin | | | A | B | B | B | A | A | B | B | B | B | A | A | C | C |

The adhesive agents of examples 1 to 10 can achieve both low adhesion to the skin and high adhesion to the substrate, have good long-term water resistance, and are guaranteed to be safe when applied to the skin.

In contrast, the adhesive agents of examples 11 and 12 did not contain a tackifier resin, and therefore did not easily adhere to SUS interfaces, resulting in inferior adhesive force to SUS steel plates. The adhesive agents of examples 13 and 14 had the hydroxyl-terminated urethane prepolymer U6 in which oxyalkylene polymers having a small number-average molecular weight were used, and therefore, regardless of the presence or absence of a tackifier resin, the adhesive agents had poor long-term water resistance and insufficient cohesion to SUS steel plates, making it impossible to obtain sufficient adhesive force.

## Claims

1. An adhesive composition comprising a hydroxyl-terminated urethane prepolymer, a tackifier resin, and a polyisocyanate compound, wherein
the hydroxyl-terminated urethane prepolymer is a reaction product of an oxyalkylene polymer having a number-average molecular weight of 3,000 or more, and a diisocyanate compound.

2. The adhesive composition according to claim 1, wherein the hydroxyl-terminated urethane prepolymer is a reaction product of an oxyalkylene polymer having a number-average molecular weight of 3,000 or more, and a diisocyanate compound in the presence of a tin-free catalyst.

3. The adhesive composition according to claim 1 or 2, wherein the tackifier resin has a softening point of 70°C or higher.

4. The adhesive composition according to claim 1 or 2, wherein the tackifier resin is a terpene-based tackifier resin or a styrene-based tackifier resin.

5. The adhesive composition according to claim 1 or 2, wherein the hydroxyl-terminated urethane prepolymer has a content of structural units based on ethylene oxide of 8 to 55% by mass.

6. The adhesive composition according to claim 1 or 2, wherein the oxyalkylene polymer comprises an oxyalkylene polymer A having the number of hydroxyl groups per molecule of 2 or more and an oxyalkylene polymer B having the number of hydroxyl groups per molecule of 1.

7. The adhesive composition according to claim 1 or 2, wherein the hydroxyl-terminated urethane prepolymer has a weight-average molecular weight of 50,000 or more.

8. A method for producing an adhesive composition by reacting an oxyalkylene polymer having a number-average molecular weight of 3,000 or more, and a diisocyanate compound to obtain a hydroxyl-terminated urethane prepolymer, and
mixing the hydroxyl-terminated urethane prepolymer, a tackifier resin, and a polyisocyanate compound.

9. The method for producing an adhesive composition according to claim 8, wherein the hydroxyl-terminated urethane prepolymer is a reaction product of an oxyalkylene polymer having a number-average molecular weight of 3,000 or more, and a diisocyanate compound in the presence of a tin-free catalyst.

10. The method for producing an adhesive composition according to claim 9, wherein the tin-free catalyst is more than 0.001 parts by mass and 0.1 parts by mass or less per 100 parts by mass of the oxyalkylene polymer.

11. An adhesive agent, being a cured product of the adhesive composition according to claim 1.

12. A patch comprising a substrate and an adhesive agent layer provided on at least a part of a surface of the substrate, wherein the adhesive agent layer comprises the adhesive agent according to claim 11.

13. The patch according to claim 12, being an adhesive tape.

14. A wearable device comprising a wearable device main body and an adhesive agent layer, wherein
the adhesive agent layer is provided on at least a part of the surface of the wearable device facing the skin and comprises the adhesive agent according to claim 11.

15. A wearable device kit comprising a wearable device main body, and at least any of the adhesive agent according to claim 11 and the patch according to claim 13.
